(19) Europäisches Patentamt

European Patent Office

Office européen des brevets

(11) **EP 1 354 595 A1**

(12) **EUROPEAN PATENT APPLICATION**

(43) Date of publication:
**22.10.2003 Bulletin 2003/43**

(51) Int Cl.7: **A61K 35/78**, A61P 37/02,
A61P 29/00, A61P 37/00,
A61P 17/06, A61P 17/00,
A61P 1/04, A61P 19/02,
A61P 13/08

(21) Application number: **03076393.2**

(22) Date of filing: **10.07.2000**

(84) Designated Contracting States:
**AT BE CH CY DE DK ES FI FR GB GR IE IT LI LU
MC NL PT SE**

(30) Priority: **09.07.1999 DK 100399
16.09.1999 DK 132399
20.09.1999 US 154651 P
16.03.2000 DK 200000434
21.03.2000 US 190919 P**

(62) Document number(s) of the earlier application(s) in
accordance with Art. 76 EPC:
**00943697.3 / 1 200 106**

(71) Applicant: **BSP Pharma A/S
2100 Copenhagen O (DK)**

(72) Inventor: **The designation of the inventor has not
yet been filed**

(74) Representative: **Plougmann & Vingtoft A/S
Sundkrogsgade 9,
P.O. Box 831
2100 Copenhagen (DK)**

(54) **Composition containing extracts of butyrospermum parkii and its use as medicament or dietary supplement**

(57)    The present invention relates to a composition comprising an extract or a concentrate of *Butyrospermum parkii* as a dietary supplement or a pharmaceutical composition and to the use of such compositions for the preparation of a medicament or a dietary supplement for the suppression of hypersensitivity and/or inflammatory reaction. The composition may optionally be formulated with a pharmaceutically acceptable carrier for systemic or topical administration. More specifically, the invention relates to a dietary supplement or a pharmaceutical composition comprising an extract or a concentrate of *Butyrospermum parkii*, wherein said extract or concentrate contains Butyrospermum-triterpenes and optionally the sterols stigmasterol, avanasterol, 24-methyl-cholest-7-enol, karitesterol A, karitesterol B and α-spinasterol.

Printed by Jouve, 75001 PARIS (FR)

**Description**

**FIELD OF THE INVENTION**

The present invention relates to a dietary supplement or a pharmaceutical composition for systemic or topical administration comprising an extract or a concentrate of *Butyrospermum parkii* optionally formulated with a pharmaceutically acceptable carrier for systemic or topical administration. More specifically, the invention relates to a dietary supplement or a pharmaceutical composition comprising an extract or a concentrate of *Butyrospermum parkii,* wherein said extract or concentrate contains the triterpene alcohols butyrospermol, lupeol, parkeol, germanicol, dammaradienol, 24-methylene-dammarenol, $\alpha$-amyrin and $\beta$-amyrin and optionally the sterols stigmasterol, avanasterol, 24-methylcholest-7-enol, karitesterol A, karitesterol B and $\alpha$-spinasterol, and to the use of such compositions for the preparation of a medicament or a dietary supplement for the suppression of hypersensitivity and/or inflammatory reaction.

**BACKGROUND OF THE INVENTION**

Hypersensitivity is defined as a state of altered reactivity in which the body reacts with an exaggerated immune response to a substance (antigen). Hypersensitivity may be caused by exogenous or endogenous antigens.

Hypersensitivity reactions underlie a large number of diseases. Among these, allergic and autoimmune conditions are of great importance. A classification of hypersensitivity diseases is given in the textbook Clinical Medicine (Kumar, P. and Clark, M.: "Clinical Medicine", 3rd edition, p. 147-150, 1994, Bailliere Tindall, London).

Type I hypersensitivity reactions (IgE mediated allergic reactions) are caused by allergens (specific exogenous antigens), e.g. pollen, house dust, animal dandruff, moulds, etc. Allergic diseases in which type I reactions play a significant role include asthma, eczema (atopic dermatitis), urticaria, allergic rhinitis and anaphylaxis.

Type II hypersensitivity reactions are caused by cell surface or tissue bound antibodies (IgG and IgM) and play a significant role in the pathogenesis of myasthenia gravis, Good-pasture's syndrome and Addisonian pernicious anaemia.

Type III hypersensitivity reactions (immune complex) are caused by autoantigens or exogenous antigens, such as certain bacteria, fungi and parasites. Diseases in which type III hypersensitivity reactions play a significant role include lupus erythematosus, rheumatoid arthritis and glomerulonephritis.

Type IV hypersensitivity reactions (delayed) are caused by cell or tissue bound antigens. This type of hypersensitivity plays a significant role in a number of conditions, e.g. graft-versus-host disease, leprosy, contact dermatitis and reactions due to insect bites.

A number of drug classes are available for the treatment of hypersensitivity reactions. Among these the corticosteroids are some of the most widely used drugs. Corticosteroids primarily exert their pharmacological action by non-selectively inhibiting the function and proliferation of different classes of immune cells resulting in suppression of hypersensitivity reactions. Unfortunately, the corticosteroids are associated with a number of serious side effects, e.g. immuno-suppression, osteoporosis and skin atrophy.

The African tree *Butyrospermum pakii,* commonly known as the Karite tree, grows wild in the dry parts of equatorial central Africa. The fruits of this tree contain a nut which is commonly known as the shea nut. The nuts are 3-4 cm long and have an oil content of approximately 50%. The major components of the oil are triglycerides, but the oil also contains several percent of an unsaponifiable fraction consisting of polyisoprenic hydrocarbons (karitene), triterpene alcohols and sterols. The oil is commonly known as shea butter. The characteristic triterpene alcohols of Butyrospermum (in the present invention termed Butyrospermum-triterpenes) are lupeol, parkeol, germanicol, dammaradienol, 24-methylene-dammarenol, butyrospermol, $\alpha$-amyrin and $\beta$-amyrin, and esters thereof, especially cinnamic acid, acetic acid or fatty acid esters.

Ordinary shea butter contains 1-5% (w/w) Butyrospermum-triterpenes and with a dosage of shea butter of 1-20% (w/w) in existing topical cosmetic or pharmaceutical products, the contents of Butyrospermum-triterpenes in such products would be maximum 1% (w/w).

FR 2770400 (WO 99/22706) discloses cosmetic or dermopharmaceutical compositions containing an extract of the flowers of *Butyrospermum parkii.* The patent does not specify the chemical components of the flowers, but to the inventors best knowledge the flowers do not contain any substantial amounts of Butyrospermum-triterpenes.

Shea butter is widely used as an emollient in cosmetic products and to a minor extent as the fatty phase of topical pharmaceutical products, such as ointments and creams.

GB 932662 discloses pharmaceutical compositions comprising butyrospermol. According to the patent butyrospermol may be obtained from *Butyrospermum parkii.*

To the inventor's best knowledge, the pharmaceutical compositions according to the invention containing extracts or concentrates of *Butyrospermum parkii* described in further detail in the following have never been disclosed before in the literature.

## SUMMARY OF THE INVENTION

It has been found by the present inventor that a composition comprising an extract or a concentrate of *Butyrospermum parkii,* said extract or concentrate comprising at least 5% of a Butyrospermum-triterpene fraction, said triterpenes being selected from the group consisting of butyrospermol, lupeol, parkeol, germanicol, dammaradienol, 24-methylene-dammarenol, $\alpha$-amyrin and $\beta$-amyrin and optionally at least one sterol selected from the group consisting of stigmasterol, avanasterol, 24-methyl-cholest-7-enol, karitesterol A, karitesterol B and $\alpha$-spinasterol wherein said Butyrospermum-triterpenes and sterols may be in the form of free alcohols or esters thereof, especially cinnamic acid, acetic acid or fatty acid esters significantly suppresses hypersensitivity reactions when used in systemic administration. Optionally, said extract or concentrate comprises a pharmaceutically acceptable carrier for systemic administration.

Furthermore, it has been found by the present inventor that a pharmaceutical composition comprising at least 5% Butyrospermum-triterpenes and optionally a pharmaceutically acceptable carrier when applied topically significantly inhibits inflammation or hypersensitivity of the skin or mucous membranes. This is surprising because such effects are not obtainable with the lower levels of Butyrospermum-triterpenes that, through the use of shea butter as an emollient, have so far been used in topical pharmaceutical or cosmetic products.

Furthermore, it has been found by the present inventor that a pharmaceutical composition containing a combination of at least 5% Butyrospermum-triterpenes and an extract of Calendula officinalis has particularly advantageous pharmacological properties.

Compared to existing therapeutic agents, such as corticosteroids or non-steroidal anti-inflammatory drugs, the pharmaceutical compositions and dietary supplements according to the present invention have the advantage of not being likely to be associated with any serious side effects, as all of their components are non-toxic and well tolerated by the organism in the pharmacologically relevant doses.

Due to the pharmacological effects mentioned above, the pharmaceutical compositions and dietary supplements according to the invention can be employed for the following therapeutic applications:

Immunomodulation.

Treatment or prevention of hypersensitivity diseases.

Treatment or prevention of inflammation or hypersensitivity of the skin.

Treatment or prevention of inflammation or hypersensitivity of mucous membranes.

Treatment or prevention of IgE mediated allergic reactions and conditions.

Treatment or prevention of autoimmune disorders.

Alleviation of pain.

Accordingly, the present invention provides a dietary supplement or a pharmaceutical composition comprising:

1. an extract or a concentrate of *Butyrospermum parkii,* said extract or concentrate comprising at least 5% of a Butyrospermum-triterpene fraction, said triterpenes being selected from the group consisting of butyrospermol, lupeol, parkeol, germanicol, dammaradienol, 24-methylene-dammarenol, $\alpha$-amyrin and $\beta$-amyrin; optionally

2. at least one sterol selected from the group consisting of stigmasterol, avanasterol, 24-methyl-cholest-7-enol, karitesterol A, karitesterol B and $\alpha$-spinasterol, wherein said triterpenes and sterols may be in the form of free alcohols or esters thereof, especially cinnamic acid, acetic acid or fatty acid esters; and optionally

3. a pharmaceutically acceptable carrier, said carrier being either suitable for systemic or topical administration.

Said pharmaceutical composition may be adapted for either systemic administration or for topical administration to the skin or mucous membrane.

Furthermore, the present invention provides the use of a composition for systemic administration comprising an extract or a concentrate of *Butyrospermum parkii* as described above and optionally a pharmaceutically acceptable carrier for systemic administration for the preparation of a medicament for immunomodulation in a mammal, for the suppression of hypersensitivity reactions in a mammal, such as IgE mediated allergic reactions, and autoimmune reactions in a mammal, and for the alleviation of pain in a mammal.

EP 1 354 595 A1

Thus, according to the invention a composition comprising an extract or a concentrate of *Butyrospermum parkii* as described above for systemic administration and optionally a pharmaceutically acceptable carrier for systemic administration can be used in a method for the treatment or prevention of a hypersensitivity disease in a mammal, said method comprising administering said composition to said mammal; and the invention comprises the use of said composition for the preparation of a medicament for the treatment or prevention of hypersensitivity diseases in a mammal.

Also, according to the invention a composition comprising an extract or a concentrate of *Butyrospermum parkii* as described above for systemic administration and optionally a pharmaceutically acceptable carrier for systemic administration can be used in a method for the treatment or prevention of an autoimmune disorder in a mammal, said method comprising administering said composition to said mammal; and the invention comprises the use of said composition for the preparation of a medicament for the treatment or prevention of autoimmune disorders in a mammal.

Further, according to the invention a composition comprising an extract or a concentrate of *Butyrospermum parkii* as described above for systemic administration and optionally a pharmaceutically acceptable carrier for systemic administration can be used in a method for the treatment or prevention of an IgE mediated allergic reaction or condition in a mammal, said method comprising administering said composition to said mammal; and the invention comprises the use of said composition for the preparation of a medicament for the treatment or prevention of IgE mediated allergic reactions and conditions in a mammal.

Also, according to the invention a composition comprising an extract or a concentrate of *Butyrospermum parkii* as described above for systemic administration and optionally a pharmaceutically acceptable carrier for systemic administration can be used in a method for the alleviation of pain in a mammal, said method comprising administering said composition to said mammal; and the invention comprises the use of said composition for the preparation of a medicament for the alleviation of pain in a mammal.

Also, the present invention provides a pharmaceutical composition, comprising: i) at least 5% (w/w) Butyrospermum-triterpenes; ii) an extract of Calendula officinalis; and optionally ii) a pharmaceutically acceptable carrier. Preferably, for topical administration to the skin or mucous membranes.

Thus, the present invention provides a pharmaceutical composition containing at least 5% Butyrospermum-triterpenes, wherein the pharmaceutical composition is formulated as a fluid, ointment, gel, liniment, emulsion (e.g. cream or lotion) or spray (e.g. aerosol).

According to the invention a pharmaceutical composition comprising at least 5% Butyrospermum-triterpenes and optionally a pharmaceutically acceptable carrier can be used in a method for the treatment or prevention of inflammation or hypersensitivity of the skin or mucous membranes in a mammal, said method comprising administering said composition topically to said mammal; and the invention comprises the use of said composition for the preparation of a medicament for the treatment or prevention of inflammation or hypersensitivity of the skin or mucous membranes in a mammal.

Also, according to the invention a pharmaceutical composition comprising at least 5% Butyrospermum-triterpenes and optionally a pharmaceutically acceptable carrier can be used in a method for the treatment or prevention of atopic dermatitis, psoriasis or contact dermatitis in a mammal, said method comprising administering said composition to said mammal; and the invention comprises the use of said composition for the preparation of a medicament for the treatment or prevention of atopic dermatitis, psoriasis or contact dermatitis in a mammal.

## DETAILED DESCRIPTION OF THE INVENTION

It has been found by the present inventor that a dietary supplement or a pharmaceutical composition comprising:

1. an extract or a concentrate of *Butyrospermum parkii,* said extract or concentrate comprising at least 5% of a Butyrospermum-triterpene fraction, said triterpenes being selected from the group consisting of butyrospermol, lupeol, parkeol, germanicol, dammaradienol, 24-methylene-dammarenol, $\alpha$-amyrin and $\beta$-amyrin, optionally

2. at least one sterol selected from the group consisting of stigmasterol, avanasterol, 24-methyl-cholest-7-enol, karitesterol A, karitesterol B and $\alpha$-spinasterol, wherein said triterpene alcohols and sterols may be in the form of free alcohols or esters thereof, especially cinnamic acid, acetic acid or fatty acid esters; and optionally

3. a pharmaceutically acceptable carrier, said carrier being suitable for either systemic or topical administration,

significantly suppresses inflammation or hypersensitivity reactions.

Said pharmaceutical composition may be adapted for either systemic administration or for topical administration to the skin or mucous membrane.

In example 1 the anti-inflammatory effect of a composition according to the invention was measured in a well-

4

established model of hypersensitivity (arthritis) in the mouse. In this experiment the composition of the invention exerted a significant effect (at 50 mg/kg) comparable to that of cyclophosphamide (at 10 mg/kg). In a separate experiment (see example 4) the $LD_{50}$ in the rat of the same composition of the invention was shown to be above 2000 mg/kg, while the $LD_{50}$ in the rat of cyclophosphamide is 94 mg/kg (The Merck Index, 13th edition, 1989, Merck and Co Inc.). Thus, the therapeutic index of the composition of the invention is far superior to that of cyclophosphamide.

When applied topically the pharmaceutical composition inhibits inflammation or hypersensitivity of the skin or mucous membranes.

In example 2 the topical anti-inflammatory effects of different compositions according to the invention are compared to an ordinary composition (control) containing shea butter corresponding to 2% Butyrospermum-triterpenes. The compositions according to the invention containing 10-30% Butyrospermum-triterpenes dose-dependently inhibit the inflammation of mouse skin, while the control has no anti-inflammatory effect. This is surprising because such effects are not obtainable with the lower levels of Butyrospermum-triterpenes that, through the use of shea butter as an emollient, have so far been used in topical pharmaceutical or cosmetic products.

The compositions of the invention for either topical or systemic administration provide a surprisingly good anti-hypersensitivity and anti-inflammatory effect with a surprisingly good safety profile. Thus, the compositions of the invention are virtually non-toxic and yet very therapeutically effective. The present inventor puts forward the hypothesis that the very beneficial therapeutic index of the compositions of the invention compared to single chemical anti-hypersensitivity drugs is due to the more complex nature of the compositions of the invention, giving a lower toxic load on the body of any single chemical compound and yet giving a surprisingly good therapeutic effect, due to synergistic effects between the components of the compositions.

More specifically, the above mentioned compositions of the invention provide the following pharmacological effects upon administration to the living organism:

Immunomodulation.

Suppression of hypersensitivity reactions.

Inhibition of inflammation or hypersensitivity of the skin. This effect can be obtained in relation to any skin disease or in relation to any disease giving rise to such symptoms of the skin, such as atopic dermatitis, psoriasis, contact dermatitis or infectious diseases.

Inhibition of inflammation or hypersensitivity of mucous membranes. This effect can be obtained in relation to any disease related to mucous membranes or in relation to any disease giving rise to such symptoms of the mucous membranes including infectious diseases.

Suppression of IgE mediated allergic reactions.

Suppression of autoimmune reactions.

Reduction of pain.

Accordingly, the present invention provides a pharmaceutical composition or a dietary supplement comprising:

1) an extract or concentrate of Butyrospermum parkii containing at least 5% (w/w) of a Butyrospermum-triterpene fraction comprising:

- at least 2% (w/w) lupeol;
- at least 2% (w/w) α-amyrin and/or β-amyrin;
- at least 2% (w/w) butyrospermol; and
- optionally at least 1% germanicol, dammaradienol, 24-methylene-dammarenol and/or parkeol,

wherein said triterpenes may be in the form of free alcohols or esters thereof, especially cinnamic acid, acetic acid or fatty acid esters; and

2) optionally a pharmaceutically acceptable carrier

Accordingly, the present invention provides a pharmaceutical composition or dietary supplement wherein the weight percentage (w/w) of the Butyrospermum-triterpene fraction in the composition typically comprises at least 5%, e.g. at least 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%, or at least 50%, such as at least 55%, e.g. at least 60%, such as at least 65%, e.g. at least 70%, at least 75%, such as at least 80%, e.g. at least 85%, such as at least 90%, e.g. at least 95%, or at least 96%, such as at least 97%, e.g. at least 98%, such as at least 99%, e.g. at least 100%; furthermore the weight percentage (w/w) of the Butyrospermum-triterpene fraction in the composition typically comprises at most 100%, e.g. 99%, at most 98%, e.g. at most 97%, e.g. at most 96, e.g. at most 95%, e.g. at most 90%, e.g. at most 85%, e.g. at most 80%, e.g. at most 75%, e.g. at most 70%, at most 65%, e.g. at most 60%, e.g. at most 55%, e.g. at most 50%, e.g. at most 45%, at most 40%, e.g. at most 35%, e.g. at most 30%, e.g. at most 25%, e.g. at most 20%, e.g. at most 15%, e.g. at most 10%, e.g. at most 9%, e.g. at most 8%, e.g. at most 7%, e.g. at most 6%, e.g. at most 5%; the weight percentage (w/w) of the Butyrospermum-triterpene fraction in the composition typically may be in the range of 5-100%, such as in the range of 6-98%, such as in the range of 7-96%, e.g. in the range of 8-94%, such as in the range of 9-92%, such as in the range of 10-90%, e.g. in the range of 12-88%, e.g. in the range of 14-86%, such as in the range of 16-84%, such as in the range of 18-82%, e.g. in the range of 20-80%

wherein the Butyrospermum-triterpene fraction is comprised of

- at least 2% (w/w) lupeol, e.g. at least 3%, such as at least 4%, e.g. at least 5%, e.g. at least 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%, or at least 50%, such as at least 55%, e.g. at least 60%, such as at least 65%, e.g. at least 70%, at least 75%, such as at least 80%, e.g. at least 85%, such as at least 90%, e.g. at least 95%, or at least 96%, such as at least 97%, e.g. at least 98%, such as at least 99%, e.g. at least 100%;
- at least 2% (w/w) $\alpha$-amyrin and/or $\beta$-amyrin, e.g. at least 3%, such as at least 4%, e.g. at least 5%, e.g. at least 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%, or at least 50%, such as at least 55%, e.g. at least 60%, such as at least 65%, e.g. at least 70%, at least 75%, such as at least 80%, e.g. at least 85%, such as at least 90%, e.g. at least 95%, or at least 96%, such as at least 97%, e.g. at least 98%, such as at least 99%, e.g. at least 100%;
- at least 2% (w/w) butyrospermol, e.g. at least 3%, such as at least 4%, e.g. at least 5%, e.g. at least 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%, or at least 50%, such as at least 55%, e.g. at least 60%, such as at least 65%, e.g. at least 70%, at least 75%, such as at least 80%, e.g. at least 85%, such as at least 90%, e.g. at least 95%, or at least 96%, such as at least 97%, e.g. at least 98%, such as at least 99%, e.g. at least 100%;
- and optionally at least 1 % (w/w) germanicol, dammaradienol, 24-methylene-dammarenol and/or parkeol, e.g. at least 2%, e.g. at least 3%, such as at least 4%, e.g. at least 5%, e.g. at least 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%, or at least 50%, such as at least 55%, e.g. at least 60%, such as at least 65%, e.g. at least 70%, at least 75%, such as at least 80%, e.g. at least 85%, such as at least 90%, e.g. at least 95%, or at least 96%, such as at least 97%, e.g. at least 98%, such as at least 99%, e.g. at least 100%.

Furthermore, the present invention provides a pharmaceutical composition or a dietary supplement comprising:

a pharmaceutical composition or a dietary supplement comprising:

i) an extract or concentrate of Butyrospermum parkii containing at least 5% (w/w) of a Butyrospermum-triterpene fraction comprising:

- 10-40% (w/w) lupeol;
- 10-40% (w/w) $\alpha$-amyrin and/or $\beta$-amyrin;
- 10-40% (w/w) butyrospermol; and
- optionally 1-30% germanicol, dammaradienol, 24-methylene-dammarenol and/or parkeol,

wherein said triterpenes may be in the form of free alcohols or esters thereof, especially cinnamic acid, acetic acid or fatty acid esters; and the Butyrospermum-triterpene fraction

Accordingly, the present invention provides a pharmaceutical composition or dietary supplement wherein the weight percentage (w/w) of the Butyrospermum-triterpene lupeol in the composition typically may be in the range of 2-95%, such as in the range of 3-95%, such as in the range of 4-90%, e.g. in the range of 5-90%, such as in the range of 5-80%, such as in the range of 5-75%, e.g. in the range of 5-70%, e.g. in the range of 5-65%, such as in the range of 5-60%, such as in the range of 5-55%, e.g. in the range of 5-50%, such as in the range of 6-45%, such as in the range of 7-40%, such as in the range of 8-40%, such as in the range of 9-40%, such as in the range of 10-40%; the weight percentage (w/w) of the Butyrospermum-triterpene α-amyrin and/or β-amyrin in the composition typically may be in the range of 2-95%, such as in the range of 3-95%, such as in the range of 4-90%, e.g. in the range of 5-90%, such as in the range of 5-80%, such as in the range of 5-75%, e.g. in the range of 5-70%, e.g. in the range of 5-65%, such as in the range of 5-60%, such as in the range of 5-55%, e.g. in the range of 5-50%, such as in the range of 6-45%, such as in the range of 7-40%, such as in the range of 8-40%, such as in the range of 9-40%, such as in the range of 10-40%; the weight percentage (w/w) of the Butyrospermum-triterpene butyrospermol in the composition typically may be in the range of 2-95%, such as in the range of 3-95%, such as in the range of 4-90%, e.g. in the range of 5-90%, such as in the range of 5-80%, such as in the range of 5-75%, e.g. in the range of 5-70%, e.g. in the range of 5-65%, such as in the range of 5-60%, such as in the range of 5-55%, e.g. in the range of 5-50%, such as in the range of 6-45%, such as in the range of 7-40%, such as in the range of 8-40%, such as in the range of 9-40%, such as in the range of 10-40%; and optionally the weight percentage (w/w) of the Butyrospermum-triterpene(s) germanicol, dammaradienol, 24-methylene-dammarenol and/or parkeol in the composition typically may be in the range of 1-95%, such as in the range of 1-90%, such as in the range of 1-80%, e.g. in the range of 1-70%, such as in the range of 1-60%, such as in the range of 2-50%, e.g. in the range of 2-40%, e.g. in the range of 2-35%, such as in the range of 2-30%.

Furthermore, the present invention provides a pharmaceutical composition especially suitable for topical administration, comprising: i) at least 5% (w/w) Butyrospermum-triterpene fraction; ii) optionally a sterol fraction; and optionally iii) a pharmaceutically acceptable carrier for topical administration, wherein

the weight percentage (w/w) of the Butyrospermum-triterpene fraction optionally together with the sterol fraction in the composition typically comprises at least 1%, e.g. at least 5%, at least 10%, at least 15%, such as at least 20%, e.g. at least 25%, e.g. at least 30%, e.g. at least 35%, such as at least 40%, or at least 45%, e.g. at least 50%, e.g. at least 55%, at least 60%, or at least 65%, such as at least 70%, e.g. at least 75%, e.g. at least 80%, e.g. at least 85%, at least 90%, such as at least 91%, e.g. at least 92%, e.g. at least 93%, e.g. at least 94%, at least 95%, or at least 96%, e.g. at least 97%, e.g. at least 98%, such as at least 99%, e.g. at least 100%; furthermore the weight percentage (w/w) of the Butyrospermum-triterpene fraction optionally together with the sterol fraction in the composition typically comprises at most 100%, e.g. 99%, at most 98%, e.g. at most 97%, e.g. at most 96, e.g. at most 95%, e.g. at most 90%, e.g. at most 85%, e.g. at most 80%, e.g. at most 75%, e.g. at most 70%, at most 65%, e.g. at most 60%, e.g. at most 55%, e.g. at most 50%, e.g. at most 45%, at most 40%, e.g. at most 35%, e.g. at most 30%, e.g. at most 25%, e.g. at most 20%, e.g. at most 15%, e.g. at most 10%. e.g. at most 9%, e.g. at most 8%, e.g. at most 7%, e.g. at most 6%, e.g. at most 5%; e.g. at most 4%; e.g. at most 3%; e.g. at most 2%; e.g. at most 1%; the weight percentage (w/w) of the Butyrospermum-triterpene fraction optionally together with the sterol fraction in the composition typically may be in the range of 1-100%, such as in the range of 2-100%, such as in the range of 3-95%, e.g. in the range of 4-90%, such as in the range of 5-80%, such as in the range of 6-75%, e.g. in the range of 7-70%, e.g. in the range of 8-65%, such as in the range of 9-60%, such as in the range of 10-55%, e.g. in the range of 10-50%, such as in the range of 10-45%, such as in the range of 10-40%.

the ratio between the Butyrospermum-triterpene(s) and the sterol(s) (Butyrospermum-triterpeneaterol) is in the range from 1:100 to 500:1, e.g. from 1:75 to 400:1, such as from 1:50 to 300:1, such as from 1:25 to 200:1, preferably from 1:20 to 100:1, e.g. from 1:10 to 75:1, such as from 1:5 to 50:1, more preferably in the range from 1:4 to 25 :1, e.g. from 1:3 to 20:1, such as from 1:2 to 19:1, e.g. from 1:1 to 18:1, e.g. from 2:1 to 17:1, such as from 3:1 to 16:1, most preferably from 4:1 to 15:1, such as from 5:1 to 14:1, e.g. from 10:1 to 13:1; and

the total weight percentage (w/w) of triterpene alcohol(s) and the sterol(s) in the composition is typically at least 0.005%, e.g. at least 0.01%, at least 0.025%, at least 0.05%, or at least 0.075%, e.g. at least 0.1%, e.g. at least 0.25%, at least 0.5%, or at least 1.0%, e.g. at least 2.0%, e.g. at least 5.0%, at least 10.0%, at least 20.0%, at least 30.0% or at least 50.0%.

In the present invention the "Butyrospermum-triterpene fraction" is defined as a fraction comprising at least one triterpene selected from the group consisting of lupeol, parkeol, germanicol, dammaradienol, 24-methylene-dammarenol, butyrospermol, α-amyrin and β-amyrin, and esters thereof, especially cinnamic acid or acetic acid esters. The compositions of the invention may contain one or more of these, such as 2, 3, 4, 5, 6, 7 or all of the Butyrospermum-triterpenes listed above, and/or 1, 2, 3, 4, 5, 6, 7 or 8 of the esters thereof, especially cinnamic acid, acetic acid or fatty acid esters, as well as mixtures comprising triterpenes as well as esters.

The "sterol fraction" is defined as a fraction comprising at least one sterol selected from the group consisting of stigmasterol, avanasterol, 24-methyl-cholest-7-enol, karitesterol A, karitesterol B and α-spinasterol. The composition may contain one or more of these, such as 2, 3, 4, 5, 6, 7 or all of the sterols listed above wherein said sterols may be in the form of free alcohols or esters thereof, especially cinnamic acid, acetic acid or fatty add esters.

The extract or concentrate of *Butyrospermum parkii* may be derived from any part of the plant, such as the fruit (nut), leaves, stem, bark or root. Preferably the extract or concentrate of the invention is derived from the fruit. Furthermore, the extract or concentrate of the invention may be derived from the oil or fat (shea butter) derived from the fruit of *Butyrospermum parki*i by any method of extraction or fractionation, e.g. comprising the unsaponifiable fraction. Preferably, the triterpene alcohols and the sterols of the invention are derived in from the unsaponifiable fraction of the oil or fat from *Butyrospermum parkii.*

Extracts or concentrates according to the invention can i.a. be obtained by extraction or distillation (e.g. hydro, steam or vacuum distillation) of fresh or dried *Butyrospermum parkii* or parts thereof, preferably the nut. Extraction may be performed with a number of different organic solvents. The extraction can be performed hot or cold by the employment of any extraction technology e.g. maceration, percolation or supercritical extraction (e.g. with carbon dioxide).

The preferred extraction solvents are acetone, methyl ethyl ketone, methyl acetate, ethyl acetate, lower alkanols having 1-4 carbon atoms, pentane, hexane, heptane and mixtures thereof. The preferred extraction temperature is close to the boiling point of the employed solvent due to extraction efficacy, but lower temperatures are also applicable, a longer period of extraction then being necessary.

By changing the composition of the applied solvent, the extraction can be made more selective for certain constituents of *Butyrospermum parkii* thus enhancing or reducing the contents thereof in the finished extract or concentrate.

After the primary extraction process, a second step of processing, such as liquid-liquid extraction, column chromatography or any type of distillation, can be employed to remove or to concentrate any constituent of the extract. Hereby any constituent of *Butyrospermum* parkii can be avoided or concentrated in the finished extract. Thus the content of any component can be standardised to obtain a composition according to the invention and the ratio between the different Butyrospermum-triterpenes may be varied dramatically in the pharmaceutical compositions of the invention and in specific cases any of the Butyrospermum-triterpenes and any number of the Butyrospermum-triterpenes may be excluded from a specific composition according to the invention. Thus, potentially a single or any other number of Butyrospermum-triterpenes may constitute the Butyrospermum-triterpene fraction of the compositions according to the invention.

"A "dietary supplement" is defined according to the U.S. Food and Drug Administration in the Dietary Supplement Health and Education Act of 1994 (DSHEA).

The DSHEA gives the following formal definition of a "dietary supplement":

"A dietary supplement:

- is a product (other than tobacco) that is intended to supplement the diet that bears or contains one or more of the following dietary ingredients: a vitamin, a mineral, an herb or other botanical, an amino acid, a dietary substance for use by man to supplement the diet by increasing the total daily intake, or a concentrate, metabolite, constituent, extract, or combinations of these things.
- is intended for ingestion in pill, capsule, tablet, or liquid form."

Similar definitions exist in other parts of the world, e.g. in Europe. Different denominations concerning "dietary supplements" are used around the world, such as "food supplements", "neutraceuticals", "functional foods" or simply "foods". In the present context the term "dietary supplement" covers any such denomination or definition.

"Systemic administration" is defined as administration by the parenteral route such as the intravenous, intraperitoneal, intraarticular, intraventricular, intracapsular, intraspinal, intramuscular, subcutaneous, intradermal, oral, buccal, sublingual, nasal, rectal, vaginal or transdermal routes.

The above mentioned pharmacological actions provide part of the rationale for the following therapeutic applications of a composition comprising an extract or a concentrate of *Butyrospermum parkii* as described above and, optionally, a pharmaceutically acceptable carrier for systemic administration:

- A method for the treatment or prevention of hypersensitivity disease or inflammation characterised by the administration of the above mentioned compositions. The therapeutic action may be relevant to all known diseases associated with hypersensitivity reactions or inflammation. Autoimmune disorders and IgE mediated allergic conditions are described below in more detail. Besides these specific therapeutic areas, the action of the above mentioned composition is relevant to all known conditions and diseases associated with hypersensitivity reaction, and the following examples are not limiting with respect to this: infections (viral, bacterial, fungal, parasitic, etc.), cold and flu, contact dermatitis, insect bites, allergic vasculitis, postoperative reactions, transplantation rejection (graft-versus-host disease), etc.

- A method for the treatment or prevention of autoimmune disorders characterised by the administration of the above mentioned compositions. The applicant puts forward the hypothesis that the therapeutic action is due to the immuno-modulating and suppressing effect on hypersensitivity reactions of the above mentioned composition. The therapeutic action may be relevant to all known autoimmune disorders and the following examples are not limiting with respect to this: Autoimmune hepatitis, Primary biliary cirrhosis, Primary sclerosing cholangitis, Autoimmune hemolytic anemias, Grave's disease, Myasthenia gravis, Type 1 Diabetes Mellitus, Inflammatory myopathies. Multiple sclerosis, Hashimoto's thyreoiditis, Autoimmune adrenalitis, Crohn's Disease, Ulcerative Colitis, Glomerulonephritis, Progressive Systemic Sclerosis (Scleroderma), Sjögren's Disease, Lupus Erythematosus, Primary vasculitis, Rheumatoid Arthritis, Juvenile Arthritis, Mixed Connective Tissue Disease, Psoriasis, Pemfigus, Pemfigoid, Dermatitis Herpetiformis, etc.

- A method for the treatment or prevention of an IgE mediated allergic reaction or condition characterised by the administration of the above mentioned compositions. The applicant puts forward the hypothesis that the therapeutic action is due to the suppressing effect on hypersensitivity reaction of the above mentioned compositions. The therapeutic action may be relevant to all known IgE mediated allergic reactions and conditions, and the following examples are not limiting with respect to this: asthma, eczema (e.g. atopic dermatitis), urticaria, allergic rhinitis, anaphylaxis, etc.

- A method for the treatment or prevention of any condition associated with pain characterised by the administration of the above mentioned compositions. The applicant puts forward the hypothesis that the therapeutic action is related to immunomodulation, possibly to a suppressing effect on hypersensitivity reactions.

Accordingly, the compositions of the invention are suitable for the treatment or prevention of diseases caused by inflammation of various tissues, e.g. inflammation of the prostate, in particular prostatitis.

"Prostatitis" is defined as inflammatory conditions affecting the prostate, including acute and chronic infections with specific bacteria and, more commonly, instances in which signs and symptoms of prostatic inflammation are present but no specific organism can be detected. Accordingly, the compositions of the invention may also be employed for the management of benign prostatic hypertrophy, a condition associated with swelling of the prostate.

Surprisingly, the present inventor has found that the therapeutic efficacy of the compositions of the invention especially for topical use may be enhanced by the addition of an extract of Calendula officinale. This is demonstrated in example 3, where the topical anti-inflammatory effect of pharmaceutical composition containing 0.1% Calendula officinalis extract and 20% Butyrospermum-triterpenes is found to be superior to a topical pharmaceutical composition containing 20% Butyrospermum-triterpenes. Thus, the present invention provides a pharmaceutical composition, comprising: i) at least 5% (w/w) Butyrospermum-triterpenes; ii) an extract of Calendula officinalis; and optionally ii) a pharmaceutically acceptable carrier, wherein

the weight percentage (w/w) of Calendula officinalis extract in the composition is typically at least 0.01%, e.g. at least 0.025%, at least 0.05%, at least 0.1%, at least 0.2%, at least 0.3%, or at least 0.4%, e.g. at least 0.5%, at least 0.75 at least 1.0%, at least 2.5%, at least 5.0%, or at least 7.5%, at least 10.0%, e.g. at least 12.5%, at least 15.0%, or at least 17.5%, at least 20.0%, e.g. at least 25.0%, at least 30.0%, at least 35.0%, or at least 40.0%, at least 50.0%, e.g. at least 75.0%; furthermore the weight percentage (w/w) of Calendula officinalis extract in the composition is typically at most 75.0%, e.g. 50.0%, at most 40.0%, e.g. at most 35.0%, e.g. at most 30.0, e.g. at most 25.0%, e.g. at most 20.0%, e.g. at most 10.0%, e.g. at most 7.5%, e.g. at most 5.0%, e.g. at most 2.5%, at most 1.0%, e.g. at most 0.75%, e.g. at most 0.5%, e.g. at most 0.4%, e.g. at most 0.3%, at most 0.2%, e.g. at most 0.1 %; the weight percentage (w/w) of Calendula officinalis extract in the composition may be in the range of 0.001-75.0%, such as in the range of 0.025-50.0%, such as in the range of 0.05-40.0%, e.g. in the range of 0.06-30%, such as in the range of 0.07-20%, such as in the range of 0.08-15%, e.g. in the range of 0.09-12%, e.g. in the range of 0.01-10%, such as in the range of 0.015-8%, such as in the range of 0.02-6%, e.g. in the range of 0.025-5%, such as in the range of 0-03-4%. such as in the range of 0.04-3%, e.g. in the range of 0.05-2%, e.g. in the range of 0.1-1%. Preferably, the pharmaceutical composition is for topical administration to the skin or mucous membranes.

The main active ingredient of the Calendula officinalis extract of the invention is faradiol. The extract may be obtained by any method of extraction, similarly to the above mentioned procedures for obtaining Butyrospermum-triterpenes and the extract may be derived from any part of the plant Calendula officinalis, preferably the leaves or the flowers.

The above mentioned pharmacological actions provide part of the rationale for the following therapeutic applications of a pharmaceutical composition for topical application according to the invention as described above:

A method for the treatment or prevention of inflammation or hypersensitivity of the skin or mucous membranes of a mammal, characterised by administering a pharmaceutical composition according to the invention to said mammal. The therapeutic action may be relevant to all known diseases associated with hypersensitivity reactions or inflammation, including autoimmune disorders and IgE mediated allergic conditions. The action of the above mentioned pharmaceutical compositions according to the invention is relevant to all known conditions and diseases associated with hypersensitivity reaction, and the following examples are not limiting with respect to this: infections (viral, bacterial, fungal, parasitic, etc.), cold and flu, contact dermatitis, insect bites, allergic vasculitis, postoperative reactions, transplantation rejection (graft-versus-host disease), asthma, eczema (e.g. atopic dermatitis), urticaria, allergic rhinitis, anaphylaxis, autoimmune hepatitis, Primary biliary cirrhosis, Primary sclerosing cholangitis, Autoimmune hemolytic anemias, Grave's disease, Myasthenia gravis, Type 1 Diabetes Mellitus, Inflammatory myopathies, Multiple sclerosis, Hashimoto's thyreoiditis, Autoimmune adrenalitis, Crohn's Disease, Ulcerative Colitis, Glomerulonephritis, Progressive Systemic Sclerosis (Scleroderma), Sjögren's Disease. Lupus Erythematosus, Primary vasculitis, Rheumatoid Arthritis, Juvenile Arthritis, Mixed Connective Tissue Disease, Psoriasis, Pemfigus, Pemfigoid, Dermatitis Herpetiformis, etc.

According to the invention the above mentioned compositions can be combined with any other active ingredient(s) to potentiate the therapeutic action.

A pharmaceutical acceptable carrier for systemic or topical administration can be water or vehicles other than water, said other vehicles can be used in the compositions and can include solids or liquids such as solvents, thickeners and powders. Examples of each of these types of vehicles, which can be used singly or as compositions of one or more vehicles, are as follows:

Emollients, such as stearyl alcohol, glyceryl monoricinoleate, glyceryl monostearate, propane-1,2-diol, butane-1,3-diol, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polyethylene glycol, triethylené glycol, lanolin, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;

solvents, such as water, methylene chloride, isopropanol, castor oil, ethylene glycol monoethyl ether, diethylene glycol monobutyl ether, diethylene glycol monoethyl ether, dimethyl sulfoxide, tetrahydrofuran, vegetable and animal oils, glycerol, ethanol, propanol, propylene glycol, and other glycols or alcohols, fixed oils;

humectants or moistening agents, such as glycerin, sorbitol, sodium 2-pyrrolidone-5-carboxylate, soluble collagen, dibutyl phthalate, gelatin;

powders, such as chalk, talc, kaolin, starch and derivatives thereof, gums, colloidal silicon dioxide, sodium polyacrylate, chemically modified magnesium aluminium silicate, hydrated aluminium silicate, carboxyvinyl polymer, sodium carboxymethyl cellulose, ethylene glycol monostearate;

gelling or swelling agents, such as pectin, gelatin and derivatives thereof, cellulose derivatives such as methyl cellulose, carboxymethyl cellulose or oxidised cellulose, cellulose gum, guar gum, acacia gum, karaya gum, tragacanth gum, bentonite, agar, alginates, carbomer, gelatine, bladderwrack, ceratonia, dextran and derivatives thereof, ghatti gum, hectorite, ispaghula husk, xanthan gum;

polymers, such as polylactic acid or polyglycolic acid polymers or copolymers thereof, paraffin, polyethylene, polyethylene oxide, polyethylene glycol, polypropylene glycol, polyvinylpyrrolidone;

surfactants, such as non-ionic surfactants, e.g. glycol and glycerol esters, macrogol ethers and esters, sugar ethers and esters, such as sorbitan esters, ionic surfactants, such as amine soaps, metallic soaps, sulfated fatty alcohols, alkyl ether sulfates, sulfated oils, and ampholytic surfactants and lecitins;

buffering agents, such as sodium, potassium, aluminium, magnesium or calcium salts (such as the chloride, carbonate, bicarbonate, citrate, gluconate, lactate, acetate, gluceptate or tartrate).

Furthermore, it is obvious that in the use according to the invention for the preparation of medicaments or dietary supplements, the above mentioned compositions may be mixed with additives such as surfactants, solvents, thickeners, stabilisers, preservatives, antioxidants, flavours, etc. to obtain a desirable product formulation suitable for systemic or topical administration. Similarly, a pharmaceutical or dietary supplement according to the invention may further contain such additives. There are no limitations on the route of administration or dosage form of the formulation, and the following examples are not limiting with respect to this: tablets, capsules, lozenges, chewing gum, fluids, granulates, sprays (e.g. aerosol), inhalants, ointments, gels, liniments, emulsions (e.g. cream or lotion), etc. Optionally, the composition may also contain surfactants such as bile salts, polyoxyethylene-sorbitan-fatty acid esters or polyalcohol mixed chain-length fatty acid esters for improving dispersibility of the composition in the digestive fluids leading to improved bioavailability or for obtaining the final dosage form of the composition.

In addition to the formulations described previously, the compositions of the invention may also be formulated as a depot preparation. Such long acting formulations may be administered by implantation (for example subcutaneously or intramuscularly) or by intramuscular injection. Thus, for example, the compositions may be formulated with suitable polymeric or hydrophobic materials (for example as an emulsion in an acceptable oil) or ion exchange resins, or as sparingly soluble derivatives, for example, as a sparingly soluble salt.

Alternatively, other pharmaceutical delivery systems may be employed. Liposomes and emulsions are well known examples of delivery vehicles that may be used to deliver compositions of the invention. Additionally, the compositions may be delivered using a sustained-release system, such as semi-permeable matrices of solid polymers containing the therapeutic agent. Various sustained-release materials have been established and are well known by those skilled in the art. Sustained-release capsules may, depending on their chemical nature, release the compositions for a few weeks up to over 100 days.

Furthermore, the invention relates to a method for the preparation of a pharmaceutically active composition as described above for systemic administration characterised by obtaining an extract or a concentrate of *Butyrospermum parkii,* said extract or concentrate comprising at least one triterpene alcohol selected from the group consisting of butyrospermol, lupeol, parkeol, germanic 01, dammaradienol, 24-methylene-dammarenol, α-amyrin and β-amyrin and at least one sterol selected from the group consisting of stigmasterol, avanasterol, 24-methyl-cholest-7-enol, karitesterol A, karitesterol B and α-spinasterol, wherein said triterpene alcohols and sterols may be in the form of free alcohols or esters thereof, especially cinnamic acid, acetic acid or fatty acid esters; and optionally mixing said extract or concentrate with a pharmaceutically acceptable carrier for systemic administration.

**EXAMPLES**

**Example 1**

Summary of the study

BPC, a concentrate of Butyrospermum parkii according to the invention, was evaluated for possible anti-inflammatory activity in BALB/c mouse arthritis induced by collagen monoclonal antibody (mAb) and lipopolysaccharide. The test substance was administered orally once daily for 3 consecutive days. Significant reduction relative to the vehicle treated control group of hind paw edema was observed at 50 mg/kg x 3 (57%, 58%, 67% and 78%) at day 7,10,14 and 17. Concurrently tested cyclophosphamide at 10 mg/kg x 3 provided a 79%, 91% and 90% reduction of hind paw edema relative to the vehicle-treated control group at day 10,14 and 17.

Test substance

A composition according to the invention was prepared by fractionation of shea butter and subsequently diluting the obtained concentrate of *Butyrospermum parkii* in corn oil. The applied concentrate of *Butyrospermum* (termed BPC in the following) contained 26% of a Butyrospermum-triterpene fraction (primarily in the form of cinnamic acid esters) selected from the group consisting of butyrospermol, lupeol, parkeol, germanicol, dammaradienol, 24-methylene-dammarenol, α-amyrin and β-amyrin, comprising the following specific Butyrospermum-triterpene amounts:

-    23% lupeol;
-    39% α-amyrin and β-amyrin; and
-    26% butyrospermol

Furthermore the concentrate contained 2.2% sterols selected from the group consisting of stigmasterol, avanasterol, 24-methyl-cholest-7-enol, karitesterol A, karitesterol B and α-spinasterol.

Dosing pattern

BPC was dissolved in 100% corn oil. The test substance was administered orally once daily for 3 consecutive days at 50 mg/kg, as well as positive control of 10 mg/kg for cyclophosphamide. Dosing volume was 5 ml/kg.

Animals

In this study, male BALB/c mice weighing 20 ± 1 grams were used. Space allocation for 5 mice was 45 x 23 x 15 cm. The animals were housed in APEC® (Allentown Gaging, Allentown, NJ 08501, U. S. A.) cages and maintained in a hygienic environment under controlled temperature (22°C - 24°C) and humidity (60% - 80%) with 12-hours light/ dark cycles for at least one week prior to being used. Free access to standard lab chow for mice and tap water was granted. All aspects of this work including housing, experimentation and disposal of animals were performed in general according to the International Guiding Principles for Biomedical Research Involving Animals (CIOMS Publication No. ISBN 92 90360194, 1985).

Chemicals

The chemicals employed in the present study were Corn Oil (Sigma, U. S. A.), Cyclophosphamide (Sigma, U. S. A.), Lipopolysaccharide (Sigma, U. S. A.), and Arthrogen-CIA™ Monoclonal Antibodies D8, F10, DI-2G and A2 (Chondrex, U. S. A.).

Equipment

Equipment employed was a Plethysmometer (Ugo Basile, U. S. A.),

Method

The study was performed according to the method of Terato et al (. Autoimmunity, 22: 137-147, 1995).
Groups of 5 BALB/c mice, 6-8 weeks of age, were used for the induction of arthritis by monoclonal antibodies (mAbs) and lipopolysaccharide (LPS). The animals were administered intravenously of a combination of 4 different mAbs (D8, F10, DI-2G and A2) in a total of 4 mg/mouse at day 0. This was followed by intravenous 25 μg of LPS 72 hours later (day 3). From day 3, test substances were administered orally once daily for 3 consecutive days. At day 5, one or two paws (particularly the hind) began to appear red and swollen, and from day 7, arthritis symptoms of the two hind paws became severely red and swollen in untreated mice. A plethysmometer (Ugo Basile Cat # 7150) with water cell (12 mm diameter) was used for the measurement of increase in both hind paw volumes at day 7, 10, 14 and 17. The percent of inhibition of increased paw volume was calculated as follows:

$$\text{Inhibition (\%): } [1 - (T_n - T_o)/(C_n - C_o)] \times 100$$

Where:

Co (Cn): Volume of day 0 (day n) in vehicle control (both hind paws summed)
To (Tn): Volume of day 0 (day n) in test compound-treated group (both hind paws summed)

Statistics

Wilcoxon rank sum test for paired differences was used for comparing swelling in the test compound treated groups with swelling in the control group.

Results

Reduction of swelling relative to the vehicle treated control group of hind paw edema was observed at 50 mg/ kg x 3 (57%, 58%, 67% and 78%) at day 7, 10, 14 and 17. The inhibition was statistically significant ($p < 0.05$, Wilcoxon) at day 10, 14 and 17. Concurrently tested cyclophosphamide at 10 mg/kg x 3 provided a statistically significant ($p < 0.05$, Wilcoxon) 79%, 91% and 90% reduction of hind paw edema relative to the vehicle-treated control group at day 10, 14 and 17.

**Example 2**

Background

Four topical pharmaceutical compositions according to the invention, containing 5-20% Butyrospermum-triterpenes, were compared to an ordinary cosmetic cream containing shea butter corresponding to 1% Butyrospermum triterpenes.
The purpose of the study was to compare the pharmacological effects of compositions according to the invention with the effects of a known composition containing shea butter in a well established assay of topical anti-inflammatory activity, phorbol ester induced inflammation in the mouse.

Methods

Four compositions according to the invention, a control composition containing shea butter and a negative control composition were prepared based on the following cream base:

| | |
|---|---|
| Hydrogenated rapeseed oil, Cremeol PS-6, Aarhus Olie, Denmark | 10.0% |
| Sodium stearoyl lactylate, Danisco Ingredients, Denmark | 5.0% |
| Sorbitan monostearate, Danisco Ingredients, Denmark | 3.0% |
| Glyceryl monostearate, Danisco Ingredients, Denmark | 2.0% |
| Methyl paraben, Unichem, Denmark | 0.3% |
| Water, purified | ad 100% |

The negative control composition was prepared without any further addition. The four pharmaceutical compositions according to the invention were prepared by the addition of a concentrate of Butyrospermum parkii (obtained by fractionation of the oil of the shea nut) corresponding to a content of Butyrospermum-triterpenes of 5%, 10%, 15% and 20%. The control shea butter composition was prepared by the addition of shea butter corresponding to 1% Butyrospermum-triterpenes.
The assay was performed according to Chang et al (Euro. J. Pharmacol. (1987)142:197). Ear inflammation was induced by topical application of phorbol ester. Groups of five BALB/c mice were pre-treated 30 minutes before phorbol ester application and 15 minutes after (post-treatment).
The degree of swelling was recorded four hours after phorbol ester application. Each mouse was its own control, as one ear was treated and one untreated.

Findings

The mean percent inhibition of ear swelling is shown in table 1. The topical pharmaceutical compositions according to the invention dose-dependently inhibited ear swelling, while the negative control and the shea butter control formulation had no effect.

Table 1

| Composition | % inhibition of ear swelling |
|---|---|
| 5% Butyrospermum-triterpene formulation | 11 |
| 10% Butyrospermum-triterpene formulation | 14 |
| 15% Butyrospermum-triterpene formulation | 20 |
| 20% Butyrospermum-triterpene formulation | 33 |
| Shea Butter Control | 0 |
| Negative Control | 0 |

Interpretation

The study clearly shows that the topical pharmaceutical compositions according to the invention containing at least 5% Butyrospermum-triterpenes possess marked anti-inflammatory effects, while an ordinary shea butter formulation has no anti-inflammatory effect.

Thus the study clearly demonstrates that a pharmaceutical composition according to the invention is pharmacologically far superior to an ordinary Shea Butter formulation.

**Example 3**

Background

Two topical pharmaceutical compositions according to the invention, one containing 20% Butyrospermum-triterpenes, the other containing 20% Butyrospermum-triterpenes and 0,1% Calendula officinalis extract were compared in a well established assay of topical anti-inflammatory activity, phorbol ester induced inflammation in the mouse.

Methods

Two compositions according to the invention and a negative control composition were prepared based on the following creme base:

| | |
|---|---|
| Hydrogenated rapeseed oil, Cremeol PS-6, Aarhus Olie, Denmark | 10.0% |
| Sodium stearoyl lactylate, Danisco Ingredients, Denmark | 5.0% |
| Sorbitan monostearate, Danisco Ingredients, Denmark | 3.0% |
| Glyceryl monostearate, Danisco Ingredients, Denmark | 2.0% |
| Methyl paraben, Unichem, Denmark | 0.3% |
| Water, purified | ad 100% |

The negative control composition was prepared without any further addition. The two pharmaceutical compositions according to the invention were prepared by the addition of a concentrate of Butyrospermum parkii (obtained by fractionation of the oil of the shea nut) corresponding to a content of Butyrospermum-triterpenes of 20% and furthermore one of them was added 0,1% Calendula officinalis extract (prepared by supercritical $CO_2$ extraction).
The assay was performed according to Chang et al (Euro. J. Pharmacol. (1987)**142**:197). Ear inflammation was induced by topical application of phorbol ester. Groups of five BALB/c mice were pre-treated 30 minutes before phorbol ester application and 15 minutes after (post-treatment).
The degree of swelling was recorded four hours after phorbol ester application. Each mouse was its own control, as one ear was treated and one untreated.

Findings

The mean percent inhibition of ear swelling is shown in table 2. The two topical pharmaceutical compositions according to the invention clearly inhibited ear swelling, while the negative control formulation had no effect.

Table 2

| Composition | % inhibition of ear swelling |
|---|---|
| Butyrospermum-triterpene + Calendula officinalis | 50 |
| Butyrospermum-triterpene | 21 |
| Negative Control | 0 |

Interpretation

The study cleanly shows that the topical pharmaceutical composition containing a combination of Butyrospermum-triterpenes and Calendula officinalis extract is superior to the composition containing Butyrospermum-triterpenes alone.

**Example 4**

Summary

BPC, a concentrate of Butyrospermum parkii according to the invention, was evaluated for acute oral toxicity in the rat. At a dose of 2000 mg/kg, BPC was found not to produce toxicity or mortality. Thus it was concluded that the $LD_{50}$ was above 2000 mg/kg body weight.

Test substance

A composition according to the invention was prepared by fractionation of shea butter and subsequently diluting the obtained concentrate of *Butyrospermum parkii* in corn oil. The applied concentrate of *Butyrospermum* (termed BPC in the following) contained 33% of a Butyrospermum-triterpene fraction comprising:

- 26% (w/w) lupeol;
- 44% (w/w) α-amyrin and/or β-amyrin; and
- 30% (w/w) butyrospermol;

Furthermore the concentrate contained 2.7% of a sterol fraction comprising:

- 43% α-spinasterol;
- 37% stigmasterol; and
- 11 % avanasterol.

Study description

The acute oral toxicity in rats was determined according to the method recommended in the OECD guideline No 420, "Acute Oral Toxicity - Fixed Dose Method", July 1992 and the EEC Directive published in: "Official Journal of the European Communities" No: L 383A, volume 35, 29.12.1992, part B1 "Acute Toxicity (Oral) - Fixed Dose Method".
The study was initiated with a sighting study, in which one female rat was given 2000 mg BPC/kg body weight. No clinical signs of toxicity were observed in this rat.
On the basis of the results of the sighting study the main study was carried out with one group consisting of 5 female rats given a dose of 2000 mg BPC/kg body weight.
All animals in the main study survived the treatment and showed no signs of evident toxicity.
The rats had a normal body weight gain during the study period.
Under the experimental conditions described in this report, it was found that the dose level tested (2000 mg BPC/kg body weight), highest required dose level, did not produce mortality. The minimal lethal dose was above 2000 mg BPC/kg body weight.

**Example 5**

Summary

A randomised, double-blind and placebo controlled phase ll clinical study is performed in patients suffering from rheumatoid arthritis to test the safety and efficacy of a concentrate of Butyrospermum parkii according to the invention.

Test substance

A composition according to the invention is prepared by fractionation of shea butter and subsequently formulating the obtained concentrate of *Butyrospermum parkii* in soft gelatine capsules each containing 750 mg of the concentrate. The applied concentrate of *Butyrospermum* (termed BPC in the following) contains 33% of a Butyrospermum-triterpene fraction comprising:

- 26% (w/w) lupeol;
- 44% (w/w) α-amyrin and/or β-amyrin; and
- 30% (w/w) butyrospermol;

Furthermore the concentrate contained 2.7% of a sterol fraction comprising:

- 43% α-spinasterol;
- 37% stigmasterol; and
- 11% avanasterol.

A placebo capsule is prepared with exactly the same appearance and weight.

Study purpose

To test the efficacy and safety of 1500 mg of BPC daily for 18 weeks, compared to placebo, in patients with rheumatoid arthritis.

Study design

Randomised, double blind, placebo controlled in two parallel groups.

Study population

40 patients, both genders, with diagnosed rheumatoid arthritis. The patients are allowed to continue with their existing medication.

Study plan

At visit 1 patients fulfilling the inclusion criteria and passing the exclusion criteria are randomised into the following groups:

- BPC 750 mg x 2 daily
- Placebo x 2 daily

At visit 1, 2 (6 weeks), 3 (12 weeks) and 4 (18 weeks) the status of the patient is evaluated using the recognised Stanford Health Assessment Questionnaire (HAQ).

**Example 6**

Summary

A randomised, double-blind and placebo controlled phase ll clinical study is performed in 15 patients suffering from psoriasis to test the safety and efficacy of a concentrate of Butyrospermum parkii according to the invention. A similar study in 120 patients suffering from atopic dermatitis using the same pharmaceutical composition according to the invention is under preparation.

Test substance

A composition according to the invention is prepared by fractionation of shea butter and subsequently formulating the obtained concentrate of *Butyrospermum parkii* in a standard cream base containing 40% of the concentrate. The components of the cream base are:
Water, PEG-6 stearate, Glycol stearate, PEG-32 stearate, Starch, Cetyl acetate og Methyl/propyl-parahydroxybenzoate.
The applied concentrate of *Butyrospermum* (termed BPC in the following) contains 33% of a Butyrospermum-triterpene fraction comprising:

- 26% (w/w) lupeol;
- 44% (w/w) α-amyrin and/or β-amyrin; and
- 30% (w/w) butyrospermol;

Furthermore the concentrate contained 2.7% of a sterol fraction comprising:

- 43% α-spinasterol;
- 37% stigmasterol; and
- 11% avanasterol.

A placebo cream (same base) is prepared with the same appearance.

Study purpose

To test the efficacy and safety of 40% BPC cream applied twice daily for 12 weeks, compared to placebo, in patients with psoriasis.

Study design

Randomised, double blind, placebo controlled left/right study (patients are their own controls).

Study population

15 patients, both genders, with diagnosed rheumatoid arthritis. The patients are allowed to continue with their existing medication.

Study plan

At visit 1 Patients fulfilling the inclusion criteria and passing the exclusion criteria are randomised into the following treatments (left/right):

- BPC 40% cream x 2 daily
- Placebo cream x 2 daily

At visit 1, 2 (4 weeks), 3 (8 weeks) and 4 (12 weeks) the status of the patient is evaluated using the recognised PASI score.


**Claims**

**1.** A pharmaceutical composition or a dietary supplement comprising:

i) an extract or concentrate of Butyrospermum parkii containing at least 5% (w/w) of a Butyrospermum-triterpene fraction comprising:

- at least 2% (w/w) lupeol;
- at least 2% (w/w) $\alpha$-amyrin and/or $\beta$-amyrin;
- at least 2% (w/w) butyrospermol; and
- optionally at least 1% germanicol, dammaradienol, 24-methylene-dammarenol and/or parkeol,

wherein said triterpenes may be in the form of free alcohols or esters thereof, especially cinnamic acid, acetic acid or fatty acid esters; and

ii) optionally a pharmaceutically acceptable carrier.

**2.** A pharmaceutical composition or a dietary supplement comprising:

i) an extract or concentrate of Butyrospermum parkii containing at least 5% (w/w) of a Butyrospermum-triterpene fraction comprising:

- 10-40% (w/w) lupeol;
- 10-40% (w/w) $\alpha$-amyrin and/or $\beta$-amyrin;
- 10-40% (w/w) butyrospermol; and
- optionally 2-30% germanicol, dammaradienol, 24-methylene-dammarenol and/or parkeol,

wherein said triterpenes may be in the form of free alcohols or esters thereof, especially cinnamic acid, acetic acid or fatty acid esters; and

ii) optionally a pharmaceutically acceptable carrier.

3.  A pharmaceutical composition or dietary supplement according to claim 1 or 2, where the extract or concentrate of Butyrospermum parkii further comprises a sterol fraction comprising at least one sterol selected from the group consisting of stigmasterol, avanasterol, 24-methyl-cholest-7-enol, karitesterol A, karitesterol B and α-spinasterol, wherein said sterols may be in the form of free alcohols or esters thereof, especially cinnamic acid, acetic acid or fatty acid esters.

4.  A pharmaceutical composition or dietary supplement according to any of the preceding claims, wherein the Butyrospermum-triterpene fraction optionally together with the sterol fraction comprises up to 100% (w/w) of the extract or concentrate of Butyrospermum parkii.

5.  A pharmaceutical composition or dietary supplement according to any of claims 3 or 4, wherein the ratio between the Butyrospermum-triterpene fraction and the sterol fraction is in the range of 1:100 to 500:1 (w/w).

6.  A pharmaceutical composition or dietary supplement according to any of the preceding claims, which further comprises an extract of Calendula officinalis.

7.  A pharmaceutical composition according to any of the preceding claims for systemic administration.

8.  A pharmaceutical composition according to any of claims 1 to 6 for topical administration, wherein the pharmaceutical composition contains at least 5% (w/w) of the Butyrospermum-triterpene fraction.

9.  A pharmaceutical composition according to claim 8, wherein the pharmaceutical composition is formulated as a fluid, ointment, gel, liniment, emulsion (e.g. cream or lotion) or spray (e.g. aerosol).

10. The use of a composition according to any of claims 1 to 9 for the preparation of a medicament or a dietary supplement for immunomodulation in a mammal.

11. The use of a composition according to any of claims 1 to 9 for the preparation of a medicament or a dietary supplement for the suppression of hypersensitivity and/or inflammatory reaction in a mammal.

12. The use of a composition according to claim 11 for the preparation of a medicament for the treatment or prevention of inflammation or hypersensitivity of the skin or mucous membranes in a mammal.

13. The use according to claim 11 or 12 for the preparation of a medicament or a dietary supplement for the treatment or prevention of autoimmune disease and/or chronic inflammatory disease in a mammal.

14. The use according to claim 13 for the preparation of a medicament or a dietary supplement for the treatment or prevention of psoriasis, atopic dermatitis, contact dermatitis, Crohn's disease, ulcerative colitis, rheumatoid arthritis or osteoarthritis in a mammal.

15. The use of a composition according to any of claims 1 to 9 for the preparation of a medicament or a dietary supplement for the alleviation of pain in a mammal.

16. The use of a composition according to any of claims 1 to 9 for the preparation of a medicament or a dietary supplement for the treatment or prevention of prostatitis and/or benign prostatic hypertrophy.

17. A method for the treatment or prevention of hypersensitivity or inflammation in a mammal, **characterised by** administering a composition according to any of claims 1 to 9 to said mammal.

18. A method for the treatment or prevention of inflammation or hypersensitivity of the skin or mucous membranes of a mammal, **characterised by** administering a composition according to any of claims 1 to 9 to said mammal.

19. A method for the treatment or prevention of an autoimmune disorder and/or a chronic inflammatory disorder in a mammal, **characterised by** administering a mixture according to any of claims 1 to 9 to said mammal.

20. A method for the treatment or prevention of psoriasis, atopic eczema, contact dermatitis, Crohn's disease, ulcerative colitis, rheumatoid arthritis and/or osteoarthritis in a mammal, **characterised by** administering a mixture according to any of claims 1 to 9 to said mammal.

21. A method for the treatment or prevention of pain in a mammal, **characterised by** administering a mixture according to any of claims 1 to 9 to said mammal.

22. A method for the treatment or prevention of prostatitis or benign prostatic hypertrophy in a mammal, **characterised by** administering a mixture according to any of claims 1 to 9 to said mammal.

23. A method for the preparation of a composition according to any of claims 1 to 9, **characterised by** obtaining an extract or a concentrate of *Butyrospermum parkii,* said extract or concentrate containing at least 5% (w/w) of a Butyrospermum-triterpene fraction comprising:

    - at least 2% (w/w) lupeol;
    - at least 2% (w/w) $\alpha$-amyrin and/or $\beta$-amyrin;
    - at least 2% (w/W) butyrospermol; and
    - optionally at least 1% germanicol, dammaradienol, 24-methylene-dammarenol and/or parkeol,

    wherein said triterpenes may be in the form of free alcohols or esters thereof, especially cinnamic acid, acetic acid or fatty acid esters; and

24. A method according to claim 22, wherein the extract or concentrate of Butyrospermum parkii further comprises a sterol fraction comprising at least one sterol selected from the group consisting of stigmasterol, avanasterol, 24-methyl-cholest-7-enol, karitesterol A, karitesterol B and $\alpha$-spinasterol, wherein said sterols may be in the form of free alcohols or esters thereof, especially cinnamic acid, acetic acid or fatty acid esters.

25. A method according to claim 22 or 23, wherein said extract or concentrate of Butyrospermum parkii is further mixed with a pharmaceutically acceptable carrier.

**European Patent**
**Office**

**PARTIAL EUROPEAN SEARCH REPORT**

**Application Number**

which under Rule 45 of the European Patent Convention EP 03 07 6393
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | GB 932 662 A (LABORATOIRES LAROCHE NAVARRON) 31 July 1963 (1963-07-31) * page 1, line 8 - page 2, line 4 * --- | 1 | A61K35/78 A61P37/02 A61P29/00 A61P37/00 A61P17/06 A61P17/00 A61P1/04 A61P19/02 A61P13/08 |
| A | FR 2 770 400 A (SEDERMA SA) 7 May 1999 (1999-05-07) ----- | | |

**TECHNICAL FIELDS SEARCHED (Int.Cl.7)**

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 29 July 2003 | Rempp, G |

EPO FORM 1503 03.82 (P04C07)

| European Patent Office | INCOMPLETE SEARCH SHEET C | Application Number EP 03 07 6393 |
|---|---|---|

Although claims 17-22,24,25 are directed to a method of treatment of the human/animal body (Article 52(4) EPC), the search has been carried out and based on the alleged effects of the compound/composition.

--------------------

Claim(s) searched completely:
        1-16,23

Claim(s) not searched:
        17-22,24,25

Reason for the limitation of the search (non-patentable invention(s)):

Article 52 (4) EPC - Method for treatment of the human or animal body by therapy

**ANNEX TO THE EUROPEAN SEARCH REPORT
ON EUROPEAN PATENT APPLICATION NO.**                    EP 03 07 6393

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2003

| Patent document cited in search report | Publication date | Patent family member(s) | Publication date |
|---|---|---|---|
| GB 932662 A | 31-07-1963 | NONE | |
| FR 2770400 A | 07-05-1999 | FR 2770400 A1<br>AU 9448498 A<br>WO 9922706 A1 | 07-05-1999<br>24-05-1999<br>14-05-1999 |

For more details about this annex : see Official Journal of the European Patent Office, No. 12/82